# EUROPÄISCHE PATENTSCHRIFT

(11) **EP 0 306 453 B1**
(45) Veröffentlichungstag und Bekanntmachung des Hinweises auf die Patenterteilung: **20.01.1993**
(21) Anmeldenummer: 88810584.8
(22) Anmeldetag: 25.08.1988
(51) Int. Cl.: C07C 311/16

(54) **Verfahren zur Herstellung von 2-(2-Chloroäthoxy)-benzosulfonamid**
Process for the preparation of 2-(2-chloroethoxy)-benzosulfon amide
Procédé pour la préparation du 2-(2-chloroéthoxy)-benzosulfonamide

(30) Priorität: 04.09.1987 US 93436; 23.05.1988 US 197480
(43) Veröffentlichungstag der Anmeldung: 08.03.1989
(73) Patentinhaber: CIBA-GEIGY AG, 4002 Basel (CH)
(72) Erfinder: Hanreich, Reinhard Georg, Dr., CH-4055 Basel (CH)

(56) Entgegenhaltungen:
- EP-A- 0 044 808
- EP-A- 0 143 627
- EP-A- 0 237 480
- DE-A- 2 623 447
- DE-A- 2 833 021
- US-A- 4 556 733

## Beschreibung

Die vorliegende Erfindung betrifft ein neues Verfahren zur Herstellung von 2-(2-Chloräthoxy)-benzosulfonamid.

Das nach dem neuen Verfahren herstellbare 2-(2-Chloräthoxy)-benzosulfonamid ist ein wertvolles Zwischenprodukt für die Synthese von herbiziden 2-(2-Chloräthoxy)-benzosulfonyl-harnstoffe. Diese Klasse von hochwirksamen Herbiziden ist in der letzten Zeit in einer grossen Anzahl von Patentanmeldungen und Publikationen beschrieben worden. In der Europäischen Patentanmeldung EP-A-44808 ist das nach dem erfindungsgemässen Verfahren herstellbare 2-(2-Chloräthoxy)-benzosulfonamid, seine Herstellung und seine Verwendung im Syntheseverfahren für die herbiziden Endprodukte aus der Klasse der Sulfonylharnstoffe beschrieben.

Die bisher beschriebenen Herstellungsverfahren für Alkoxybenzosulfonamide sind für grosstechnische Anwendung weniger geeignet, weil entweder instabile Diazoniumsalze als Zwischenprodukte erzeugt werden und die Austauschreaktion vom Sandmeyer-Typus mit Cu(I)-Verbindungen nur einen unzureichenden Grad an Selektivität besitzt, oder weil Produktgemische entstehen, die aufwendige Trennungsmethoden erfordern.

Es besteht somit ein Bedürfnis nach einem kostengünstigen, im grosstechnischen Massstab durchführbaren Verfahren zur Herstellung von 2-(2-Chloräthoxy)-benzosulfonamid.

Ueberraschenderweise befriedigt das erfindungsgemässe neue Verfahren dieses Bedürfnis weitgehend.

Gemäss vorliegender Erfindung wird vorgeschlagen, das 2-(2-Chloräthoxy)-benzosulfonamid der Formel I 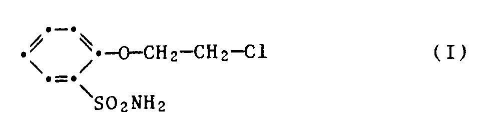
in der Weise herzustellen, dass man ein 4-Chlorphenol der Formel II 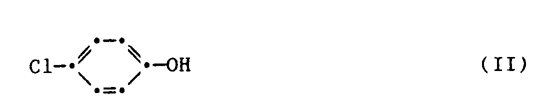
mit Aethylencarbonat zum 4-(2-Hydroxyäthoxy)-chlorbenzol der Formel III 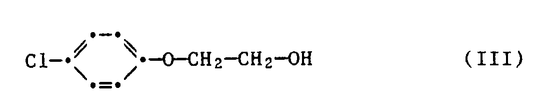
veräthert, mit Phosgen oder Thionylchlorid zum 4-(2-Chloräthoxy)-chlorbenzol der Formel IV 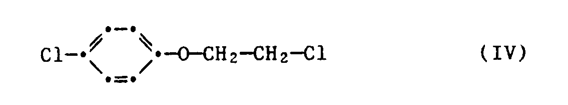
chloriert, mit Chlorsulfonsäure ClSO₃H und Natriumhydroxid in das Natriumsulfonat der Formel V 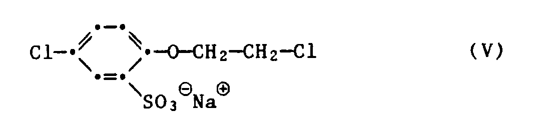
überführt, dieses zur Verbindung der Formel VI 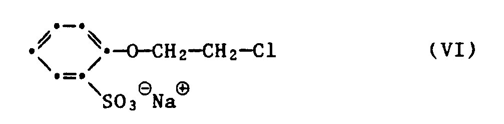
hydriert, welche anschliessend mit Phosgen in das Sulfonsäurechlorid der Formel VII 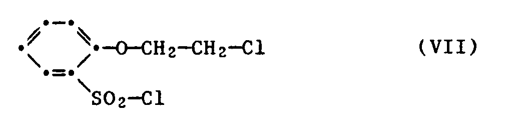
überführt wird, welches mit Ammoniak zum Sulfonamid der Formel I umgesetzt wird.

Das durch das erfindungsgemässe Verfahren hergestellte 2-(2-Chloräthoxy)-benzosulfonamid weist eine hohe Reinheit auf.

Es kann in an sich bekannter Weise direkt durch Reaktion mit einem geeigneten Pyrimidinyl- oder Triazinylcarbamat oder einem entsprechenden Isocyanat zu einem Pflanzenwirkstoff aus der Klasse der Sulfonylharnstoffe umgesetzt werden. Als Alternative zu diesem Verfahren überführt man das 2-(2-Chloräthoxy)-benzosulfonamid der Formel I zunächst in ein entsprechendes Isocyanat oder Carbamat und setzt dieses mit geeigneten Pyrimidinyl- oder Triazinylaminen zu den pflanzenwirksamen Sulfonyl-harnstoffen um.

Die Ausgangsverbindung der Formel II ist bekannt und im Handel erhältlich.

Zur Durchführung der ersten beiden Schritte (II → III → IV) des erfindungsgemässen Verfahrens verwendet man handelsübliches Aethylencarbonat (1,3-Dioxolan-2-on) sowie Phosgen oder Thionylchlorid. Beide Reaktionsschritte können im gleichen Gefäss durchgeführt werden, eine Isolierung des Zwischenproduktes der Formel III ist dabei nicht notwendig. Ferner ist als bemerkenswert hervorzuheben, dass in diesen beiden Reaktionsschritten das Ausgangsmaterial der Formel II sowie Aethylencarbonat und Phosgen, bzw. Thionylchlorid, in reiner Form ohne Verwendung eines Lösungsmittels eingesetzt werden können. Für den ersten Schritt (II → III) liegt die Reaktionstemperatur im Bereich von +130°C bis +150°C. Vorzugsweise wird dieser Reaktionsschritt in Gegenwart einer katalytischen Menge eines tertiären Amins wie zum Beispiel Tributylamin oder 1,8-Diazabicyclo[5.4.0]undec-7-en durchgeführt. Die Reaktionstemperatur für den zweiten Schritt (III → IV) liegt im Bereich von +70°C bis +90°C. Vorzugsweise wird dieser Reaktionsschritt in Gegenwart einer katalytischen Menge von Dimethylformamid oder Dimethylacetamid durchgeführt. Bevorzugt ist dabei Dimethylformamid.

Nach Durchführung der Reaktionsfolge II → III → IV in einem "Eintopfverfahren" wird das Rohprodukt durch Zusatz verdünnter Natronlauge und anschliessende Destillation der Verbindung der Formel IV aufgearbeitet. Durch diese Methode wird das Zwischenprodukt der Formel IV in hoher Reinheit erhalten.

Zur Durchführung des dritten Schrittes (IV → V) des erfindungsgemässen Verfahrens verwendet man handelsübliche Chlorsulfonsäure. Zur Umsetzung werden pro Mol der Verbindung IV eine äquimolare Menge der Chlorsulfonsäure eingesetzt. Die Reaktionstemperaturen bei diesem Verfahrensschritt liegen im Bereich von -20°C bis +60°C, wobei ein Bereich von -10°C bis +5°C bevorzugt ist. Nach Aufarbeiten der Reaktionsmischung durch Neutralisation mit wässrigem Natriumhydroxid bei einer Temperatur von +50°C bis +90°C, vorzugsweise von +60°C bis +80°C, wird das entsprechende Natriumsulfonat der Formel V erhalten. In einem bevorzugten Ausführungsform der Aufarbeitung wird die Reaktionsmischung nach Verdünnen mit Wasser mit Toluol oder Xylol bei einer Temperatur von 0°C bis +50°C, vorzugsweise von +20°C bis +40°C, extrahiert. Die verbleibende wässrige Lösung wird bei einer Temperatur von +50°C bis +70°C mit Natriumhydroxid neutralisiert.

Mit Vorteil wird diese Reaktion in einem inerten Lösungsmittel wie einem Alkan oder Chloralkan durchgeführt. Lösungsmittel der oben genannten Art sind Pentan, Hexan, Heptan, Oktan, Dekan, Dodecan, aber auch Cyclopentan, Cyclohexan oder Dekalin, sowie Methylenchlorid, Chloroform, Tetrachlorkohlenstoff, Dichloräthan, Trichloräthan oder Tetrachloräthan. Bevorzugt sind Cyclohexan und n-Dekan.

Die Dechlorierung der Verbindung der Formel V zur Verbindung der Formel VI durch katalytische Hydrierung wird im allgemeinen unter milden Bedingungen bei Temperaturen von +20°C bis +70°C, vorzugsweise von +40°C bis +60°C, bei einem Druck von 1 bis 5 bar, vorzugsweise 1 bis 4 bar, in einem inerten Lösungsmittel in Gegenwart eines säurebindenden Mittels mit Wasserstoff durchgeführt. Als Katalysatoren werden im wesentlichen Edelmetallkatalysatoren verwendet wie Platin in Form von Platinoxid oder Palladium, Platinmoor oder Platin auf Bariumsulfat, Palladiummoor oder Palladium auf Kohle. Am breitesten einsetzbar ist Palladium auf Kohle in handelsüblicher Form als 5 % Palladium/Kohle. Als säurebindendes Mittel wird üblicherweise Natriumhydroxid verwendet. Diese Base wird während der Reaktion kontinuierlich dem Reaktionsgemisch zugefügt, um den pH-Wert konstant zu halten; hier zwischen pH 9,5 und pH 11,5. In der bevorzugten Ausführungsform wird die Verbindung der Formel V unter Normaldruck bei +40°C bis +60°C in Gegenwart eines 5 %igen Palladium/ Kohle-Katalysators in einem Wasser-Base-Gemisch mit Wasserstoff hydriert.

Nach Abschluss der Hydrierung wird der Katalysator durch Filtration oder Sedimentation aus dem Reaktionsgemisch abgetrennt. In einer bevorzugten Ausführungsform der Reaktion wird das Filtrat durch Zusatz von Chlorbenzol und Abdestillieren des azeotropen Gemisches getrocknet. In diesem bevorzugten Verfahren wird das als Destillationsrückstand erhaltene 2-(2-Chloräthoxy)-benzosulfonsäure-Natriumsalz direkt in den nachfolgenden Reaktionsschritt (VI → VII) überführt.

Die Ueberführung der Verbindung der Formel VI in das Sulfonylchlorid der Formel VII erfolgt durch Umsatz mit gasförmigem Phosgen. Aus ökonomischen und ökologischen Gründen wird das Phosgen vorzugsweise in einem Phosgengenerator kontinuierlich ohne Zwischenlagerung erzeugt. Vorteilhaft ist bei der Umsetzung die Verwendung eines Katalysators wie zum Beispiel Dimethylformamid oder Dimethylacetamid. Die Ueberführung des Natriumsulfonats in das Sulfonylchlorid (VI →VII) geschieht unter den für diesen Verfahrenstyp üblichen Reaktionsbedingungen. Wesentliche Merkmale dabei sind die Vermeidung von Feuchtigkeit und die Inertheit des Lösungsmittels. Die oben beschriebenen Reaktionsbedingungen entsprechen diesen Anforderungen. Bevorzugte Reaktionstemperaturen liegen im Bereich von +60°C bis +120°C, wobei eine Temperatur von etwa 95°C besonders bevorzugt ist. In einer bevorzugten Ausführungsform des Verfahrens wird der in der Lösung vorhandene Ueberschuss an Phosgen durch Waschen mit Wasser entfernt. Die organische Phase wird filtriert und direkt in den nächsten Reaktionsschritt (VII → I) überführt.

Die Ueberführung des Sulfonsäurechlorids der Formel VII in das entsprechende Sulfonamid der Formel I erfolgt unter für diesen Reaktionstyp an sich bekannten Bedingungen, zum Beispiel durch Zusatz von wässrigem Ammoniak zur Verbindung der Formel VII unter Normaldruck bei einer Temperatur zwischen 0°C und +100°C, vorzugsweise bei +50°C bis +70°C. Bevorzugt wird die Reaktion mit einer wässrigen Lösung von Ammoniak durchgeführt, welche der nach dem Chlorierungsschritt (VI → VII) erhaltenen Sulfonsäurechloridlösung zugesetzt wird.

Nach Abschluss der Amidierungsreaktion (VII →I) wird die Wasser und überschüssiges Ammoniak enthaltende wässrige Phase azeotrop bei +70°C und 80 mbar destilliert. Die erhaltene Suspension wird bei +100°C über Aktivkohle filtriert. Nach Abkühlen des Filtrates auf +10°C kristallisiert das Sulfonamid der Formel I in 70 % Ausbeute [berechnet auf 4-Chlor-2-(chloräthoxy)-benzol] bei einem Reinheitsgrad von 98-99 % aus.

In einer bevorzugten Ausführungsform des erfindungsgemässen Verfahrens setzt man die Verbindung der Formel IV zu der Verbindung der Formel I um, ohne die Zwischenprodukte der Formeln V, VI und VII zu isolieren.

In dieser bevorzugten Ausführungsform setzt man die Verbindung der Formel IV bei einer Temperatur zwischen -10°C und +5°C in n-Dekan oder Hexan mit einer äquimolaren Menge Chlorsulfonsäure um, nimmt die Mischung mit Wasser auf, extrahiert mit Xylol, neutralisiert die Reaktionsmischung mit wässrigem Natriumhydroxid bei +50°C bis +70°C, dechloriert mit Wasserstoff in Gegenwart eines Säureakzeptors bei einem Druck von 1 bis 5 bar und einer Temperatur von +20°C bis +70°C in Gegenwart eines 5%igen Palladium/ Kohle-Katalysators, überführt in das Sulfonsäurechlorid durch Umsetzen der Reaktionsmischung mit Phosgen bei einer Temperatur zwischen +60°C und +120°C und erzeugt das Endprodukt durch anschliessendes Behandeln der Reaktionsmischung mit wässriger Ammoniaklösung.

Eine besonders bevorzugte Ausführungsform des erfindungsgemässen Verfahrens ist dadurch gekennzeichnet, dass als einziges Zwischenprodukt die Verbindung der Formel IV isoliert wird, das heisst die Reaktion der Verbindung der Formel II zu der Verbindung der Formel I verläuft ohne eine Isolierung der Zwischenprodukte der Formeln III, V, VI und VII.

In einer ganz besonders bevorzugten Ausführungsform des erfindungsgemässen Verfahrens setzt man die Verbindung der Formel II mit Aethylencarbonat in Gegenwart einer katalysierten Menge an Tributylamin bei einer Temperatur von +130°C bis +150°C um, behandelt die erhaltene Mischung mit Phosgen oder Thionylchlorid bei einer Temperatur von +70°C bis +90°C in Gegenwart einer katalytischen Menge an Dimethylformamid wobei man die Verbindung der Formel IV erhält, welche nach erfolgter Reinigung mit einer äquimolaren Menge Chlorsulfonsäure in Hexan oder n-Dekan bei einer Temperatur zwischen -10°C und +5°C umgesetzt, in Wasser aufgenommen, mit Xylol extrahiert, bei einer Temperatur von +50°C bis +70°C mit wässrigem Natriumhydroxid neutralisiert, mit Wasserstoff in Gegenwart eines säurebindenden Mittels bei einem Druck von 1 bis 5 bar und einer Temperatur von +20°C bis +70°C in Gegenwart eines 5%igen Palladium/Kohle-Katalysators dechloriert wird und überführt in das Sulfonsäurechlorid durch Umsetzen der Reaktionsmischung mit Phosgen bei einer Temperatur zwischen +60°C und +120°C und erzeugt das Endprodukt durch anschliessendes Behandeln der Reaktionsmischung mit wässriger Ammoniaklösung.

In der am meisten bevorzugten Ausführungsform des erfindungsgemässen Verfahrens setzt man die Verbindung der Formel II mit Aethylencarbonat in Gegenwart einer katalytischen Menge an Tributylamin bei einer Temperatur von +130°C bis +150°C um, überführt mit Thionylchlorid bei einer Temperatur von +70°C bis +90°C in Gegenwart einer katalytischen Menge an Dimethylformamid in die Verbindung der Formel IV, die nach erfolgter Reinigung mit einer äquimolaren Menge Chlorsulfonsäure in n-Dekan bei einer Temperatur von -10°C bis +5°C umgesetzt, mit Wasser aufgenommen, mit Xylol extrahiert, mit wässrigem Natriumhydroxid bei einer Temperatur von +50°C bis +70°C neutralisiert wird und dechloriert mit Wasserstoff in Gegenwart von Natriumhydroxid bei einem Druck von 1 bis 4 bar und einer Temperatur von +40°C bis +60°C in Gegenwart eines 5%igen Palladium/Kohle-Katalysators, überführt in das Sulfonsäurechlorid durch Umsetzen der Reaktionsmischung mit Phosgen bei einer Temperatur von +95°C und erzeugt das Endprodukt durch anschliessendes Behandeln der Reaktionsmischung mit wässriger Ammoniaklösung bei einer Temperatur von +60°C.

### Herstellungsbeispiele

### Beispiel H1: 4-Chlor-(2-chloräthoxy)-benzol

26,4 g 4-Chlorphenol und 18,7 g Aethylencarbonat werden nach Zugabe von 1 g Tributylamin langsam auf +150°C erhitzt. Die Reaktionsmischung wird etwa 3 Stunden bei +150°C gehalten bis die Kohlendioxidentwicklung zum Stillstand gekommen ist. Nach Abkühlen auf +85°C und Zufügen von 1 g Dimethylformamid wird 24 g gasförmiges Phosgen in die Reaktionsmischung eingeleitet. Die Reaktionstemperatur wird für 8 Stunden bei +85°C gehalten und dann auf +60°C erniedrigt. Nach Beseitigung des Phosgenüberschusses durch Hinzufügung von 100 ml Wasser wird die Reaktionsmischung in Gegenwart eines 5%igen Palladium/Kohle-Katalysators durch Zusetzen von 10,5 g einer 25%igen wässrigen Lösung von Natriumhydroxid neutralisiert. Die organische Phase wird abgetrennt und destilliert. Man erhält 36,0 g (93 % der Theorie) 4-Chlor-(2-chloräthoxy)-benzol, Sdp. 150-152°C/40 mb.

### Beispiel H2: 4-Chlor-(2-chloräthoxy)-benzol

Nach Zusatz von 112,4 g Tributylamin zu einer Mischung von 778,8 g 4-Chlorphenol und 560 g Aethylencarbonat wird die erhaltene Mischung langsam auf +150°C erhitzt und etwa 3 Stunden bei dieser Temperatur gehalten bis kein Kohlendioxid mehr entwickelt wird. Nachdem die Hauptmenge des Tributylamins bei +95°C bis +105°C und 35 mbar aus dem Rohprodukt abdestilliert worden ist, wird das erhaltene 4-(2-Hydroxyäthoxy)-chlorbenzol in Gegenwart von 13,2 g Dimethylformamid bei +85°C bis +90°C tropfenweise innerhalb von 4 Stunden mit 785,4 g Thionylchlorid versetzt, wobei eine kräftige Chlorwasserstoff-und Schwefeldioxidgas-Entwicklung zu beobachten ist. Nach beendeter Zugabe wird die Reaktionsmischung mit 500 ml Wasser bei +30°C bis +50°C aufgenommen und das erhaltene Zweiphasensystem mit 384 g 30%iger wässriger Natriumhydroxidlösung bei +50°C bis +60°C neutralisiert. Die organische Phase wird abgetrennt und bei +87°C bis +95°C und 5 mbar destilliert. Man erhält 1044 g (90 % der Theorie) 4-Chlor-(2-chloräthoxy)-benzol.

### Beispiel H3: 2-(2-Chloräthoxy)-benzosulfonamid

a) Zu einer Lösung von 203 g 4-Chlor-(2-chloräthoxy)-benzol in 230 ml n-Dekan werden innerhalb von 2 Stunden bei einer Temperatur von +35°C bis +40°C 124,7 g Chlorsulfonsäure zugesetzt. Während der Zugabe entwickelt sich Chlorwasserstoff und ein grober Niederschlag wird abgeschieden. Nachdem die erhaltene Suspension weitere 2 Stunden bei +35°C bis +40°C gehalten worden ist, wird die Reaktionsmischung mit 400 ml Wasser aufgenommen und anschliessend bei einer Temperatur von +60°C bis +65°C mit 158 g einer 30%igen wässrigen Natriumhydroxidlösung neutralisiert. Die wässrige Phase (885 g) wird heiss abgetrennt und direkt in die anschliessende Hydrierung eingesetzt.
b) In einem Kolben mit Rührwerk werden bei +40°C bis +45°C 15 g eines 5%igen Palladium/Kohle-Katalysators zu der oben erhaltenen Natriumsalzlösung (885 g) zugesetzt. Diese Mischung wird bei Normaldruck bei einer Temperatur von +40°C bis +60°C mit Wasserstoff hydriert. Durch kontinuierliche Zugabe von 126,5 g einer 30%igen wässrigen Natriumhydroxidlösung wird der pH-Wert dabei zwischen pH 9,5 und pH 11,5 gehalten. Wenn keine Wasserstoff-Absorption mehr beobachtet werden kann, wird der Palladiumkatalysator abfiltriert. Das Filtrat wird in 750 ml Chlorbenzol aufgenommen und das enthaltene Wasser wird durch azeotrope Destillation entfernt. Es werden 1150 g einer Suspension erhalten, die das Natriumsalz der 2-(2-Chloräthoxy)-benzosulfonsäure enthält.
c) 1150 g der oben erhaltenen chlorbenzolischen Suspension werden mit 600 ml Chlorbenzol verdünnt und auf +90°C erhitzt. Nach Zusatz von 6,8 g Dimethylformamid werden 120 g (1,2 Mol) gasförmiges Phosgen in die Mischung eingeleitet. Nachdem die Reaktionsmischung für 2 bis 3 Stunden bei +90°C gehalten wurde, wird die Reaktion durch Zusatz von 1200 ml Wasser bei einer Temperatur von +70°C gestoppt. Die untere, organische Phase wird bei +60°C bis +65°C abgetrennt und mit 160 g einer 30%igen wässrigen Ammoniaklösung umgesetzt. Nach beendeter Zugabe der Ammoniaklösung wird die erhaltene Suspension eine Stunde lang bei +60°C gehalten und anschliessend bei +100°C zum Entfernen der anorganischen Salze über Aktivkohle filtriert. Das im Filtrat vorhandene Wasser wird durch azeotrope Destillation entfernt. Das Auskristallisieren des Produktes wird durch Abkühlen der Chlorbenzollösung auf +10°C erreicht. Nach Abfiltrieren des farblosen, kristallinen Niederschlages und Trocknen im Vakuum erhält man 170 g (70 % der Theorie) 2-(2-Chloräthoxy)-benzosulfonamid mit einem Schmelzpunkt von 117°C bis 118°C.

### Beispiel H4: 2-(2-Chloräthoxy)-benzosulfonamid

In der Herstellungsmethode gemäss Beispiel H3 kann der erste Reaktionsschritt (a) durch folgende Arbeitsweise ersetzt werden:
Zu einer Suspension von 203 g 4-Chlor-(2-chloräthoxy)-benzol in 380 g n-Dekan werden bei einer Temperatur von -10°C bis +5°C innerhalb von 2 Stunden 123,8 g Chlorsulfonsäure zugesetzt. Nach beendeter Chlorwasserstoffentwicklung wird die erhaltene Suspension für weitere 2 Stunden bei -10°C bis +5°C gehalten. Nach dem Aufnehmen der Reaktionsmischung mit 400 ml Wasser wird mit 300 ml Xylol bei +20°C bis +40°C extrahiert. Die wässrige Phase wird abgetrennt und mit 158 g einer 30%igen wässrigen Natriumhydroxidlösung bei einer Temperatur von +60°C bis +65°C neutralisiert. Die wässrige Phase (885 g) wird heiss abgetrennt und direkt für die Hydrierung eingesetzt.

Die Reaktionsschritte (b) und (c) werden wie im Beispiel H3 durchgeführt.

## Patentansprüche

1. Verfahren zur Herstellung von 2-(2-Chloäthoxy)-benzosulfonamid der Formel I 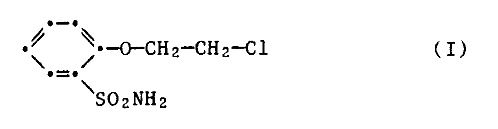 dadurch gekennzeichnet, dass man ein 4-Chlorphenol der Formel II 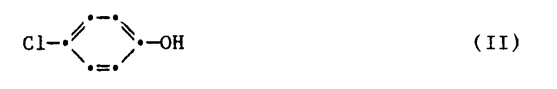 mit Aethylencarbonat veräthert, das entstandene 4-(2-Hydroxyäthoxy)-chlorbenzol der Formel III 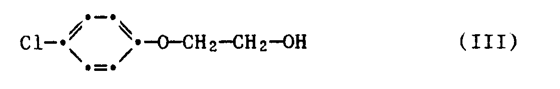 mit Phosgen oder Thionylchlorid zum 4-(2-Chloräthoxy)-chlorbenzol der Formel IV 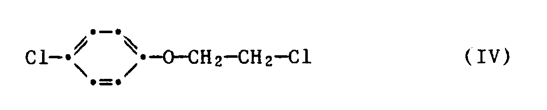 chloriert, mit Chlorsulfonsäure ClSO₃H und Natriumhydroxid in das Natriumsulfonat der Formel V 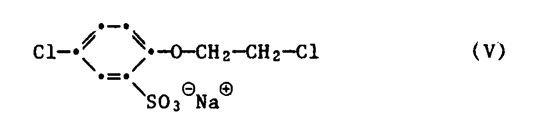 überführt, dieses zur Verbindung der Formel VI 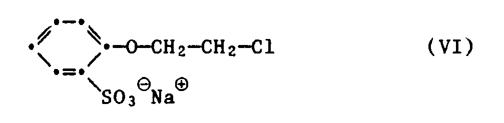 hydriert, welche anschliessend mit Phosgen in das Sulfonsäurechlorid der Formel VII 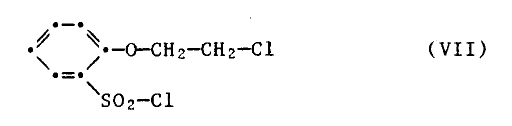 überführt wird, welches mit Ammoniak zum Sulfonamid der Formel I umgesetzt wird.

2. Verfahren gemäss Anspruch 1, dadurch gekennzeichnet, dass man die Reaktion der Verbindung der Formel II zu der Verbindung der Formel IV ohne Isolierung des Zwischenproduktes der Formel III durchführt, indem man die Verbindung der Formel II zuerst mit Aethylencarbonat und anschliessend die erhaltene Reaktionsmischung mit Phosgen oder Thionylchlorid behandelt.

3. Verfahren gemäss Anspruch 2, dadurch gekennzeichnet, dass man die Verbindung der Formel II mit Aethylencarbonat in Gegenwart einer katalytischen Menge an Tributylamin bei einer Temperatur von +130°C bis +150°C umsetzt und die erhaltene Reaktionsmischung mit Phosgen oder Thionylchlorid in Gegenwart einer katalytischen Menge Dimethylformamid bei einer Temperatur von +70°C bis +90°C behandelt.

4. Verfahren gemäss Anspruch 1, dadurch gekennzeichnet, dass man den Umsatz der Verbindung der Formel IV zur Verbindung der Formel V bei einer Temperatur von -20°C bis +60°C mit einer äquimolaren Menge Chlorsulfonsäure in Hexan oder n-Dekan durchgeführt und die Lösung bei einer Temperatur zwischen +50°C und +90°C mit wässrigem Natriumhydroxid neutralisiert.

5. Verfahren gemäss Anspruch 4, dadurch gekennzeichnet, dass man den Umsatz der Verbindung der Formel IV zur Verbindung der Formel V bei einer Temperatur von -10°C bis +5°C mit einer äquimolaren Menge an Chlorsulfonsäure in Hexan oder n-Dekan durchführt, die Lösung mit Wasser aufnimmt, mit Xylol oder Toluol extrahiert und anschliessend bei einer Temperatur von +50°C bis +70°C mit wässrigem Natriumhydroxid extrahiert.

6. Verfahren gemäss Anspruch 1, dadurch gekennzeichnet, dass man bei der Ueberführung der Verbindung der Formel V in die Verbindung der Formel VI die Dechlorierung mit Wasserstoff bei einem Druck von 1 bis 5 bar und einer Temperatur von +20°C bis +70°C in Gegenwart eines 5%igen Palladium/Kohle-Katalysators in einer Mischung aus Base und Wasser durchführt.

7. Verfahren gemäss Anspruch 1, dadurch gekennzeichnet, dass man das Natriumsulfonat der Formel VI bei einer Temperatur von +60°C bis +120°C durch Umsetzen mit Phosgen in das Sulfonsäurechlorid der Formel VII überführt.

8. Verfahren gemäss Anspruch 1, dadurch gekennzeichnet, dass man das Sulfonsäurechlorid der Formel VII mit einer wässrigen Ammoniaklösung in Gegenwart von Chlorbenzol zur Verbindung der Formel I umsetzt.

9. Verfahren gemäss Anspruch 1, dadurch gekennzeichnet, dass man den Umsatz der Verbindung der Formel IV zu der Verbindung der Formel I ohne Isolierung der Zwischenprodukte der Formeln V, VI und VII durchführt.

10. Verfahren gemäss Anspruch 1, dadurch gekennzeichnet, dass man den Umsatz der Verbindung der Formel II zu der Verbindung der Formel I ohne Isolierung der Zwischenprodukte der Formeln III, V, VI und VII durchführt.

11. Verfahren gemäss Anspruch 9, dadurch gekennzeichnet, dass man die Verbindung der Formel IV mit einer äquimolaren Menge oder einem leichten Ueberschuss Chlorsulfonsäure in Cyclohexan oder n-Dekan bei einer Temperatur von +20°C bis +60°C umsetzt, bei einer Temperatur von +50°C bis +90°C mit wässrigem Natriumhydroxid neutralisiert, mit Wasserstoff in Gegenwart eines säurebindenden Mittels bei einem Druck von 1 bis 5 bar und einer Temperatur von +20°C bis +70°C in Gegenwart eines 5%igen Palladium/ Kohle-Katalysators dechloriert, durch Umsatz der Mischung mit Phosgen bei einer Temperatur von +60°C bis +120°C in das Sulfonsäurechlorid überführt und anschliessend das Endprodukt durch Umsetzung der Reaktionsmischung mit wässriger Ammoniaklösung erzeugt.

12. Verfahren nach Anspruch 11, dadurch gekennzeichnet, dass man die Verbindung der Formel IV mit einer äquimolaren Menge an Chlorsulfonsäure in Hexan oder n-Dekan bei einer Temperatur von -10°C bis +5°C umsetzt, mit Wasser aufnimmt, mit Xylol extrahiert, mit wässrigem Natriumhydroxid bei einer Temperatur von +50°C bis +70°C neutralisiert, mit Wasserstoff in Gegenwart eines Säureakzeptors bei einem Druck von 1 bis 5 bar und einer Temperatur von +20°C bis +70°C in Gegenwart eine 5%igen Palladium/Kohle-Katalysators dechloriert, durch Umsatz der Mischung mit Phosgen bei einer Temperatur von +60°C bis +120°C in das Sulfonsäurechlorid überführt und anschliessend durch Umsetzung der Reaktionsmischung mit wässriger Ammoniaklösung das Endprodukt zu erzeugt.

13. Verfahren nach Anspruch 1, dadurch gekennzeichnet, dass man die Verbindung der Formel II in Gegenwart einer katalytischen Menge Tributylamin bei einer Temperatur von +130°C bis +150°C mit Aethylencarbonat umsetzt, durch Umsatz der erhaltenen Mischung mit Phosgen oder Thionylchlorid in Gegenwart einer katalytischen Menge Dimethylformamid bei einer Temperatur von +70°C bis +90°C in die Verbindung der Formel IV überführt, diese nach Reinigung mit einer äquimolaren Menge Chlorsulfonsäure in Hexan oder n-Dekan bei einer Temperatur von -10°C bis +5°C umsetzt, mit wässrigem Natriumhydroxid bei einer Temperatur von +50°C bis +90°C neutralisiert, mit Wasserstoff in Gegenwart eines Säureakzeptors bei einem Druck von 1 bis 5 bar und einer Temperatur von +20°C bis +70°C in Gegenwart eines 5%igen Palladium/Kohle-Katalysators dechloriert, durch Umsatz der Mischung mit Phosgen bei einer Temperatur von +60°C bis +120°C in das Sulfonsäurechlorid überführt und anschliessend durch Umsetzung der Reaktionsmischung mit wässriger Ammoniaklösung das Endprodukt erzeugt.

14. Verfahren nach Anspruch 13, dadurch gekennzeichnet, dass man die Verbindung der Formel II in Gegenwart einer katalytischen Menge Tributylamin bei einer Temperatur von +130°C bis +150°C mit Aethylencarbonat umsetzt, durch Umsatz der erhaltenen Mischung mit Phosgen oder Thionylchlorid in Gegenwart einer katalytischen Menge Dimethylformamid bei einer Temperatur von +70°C bis +90°C in die Verbindung der Formel IV überführt, diese nach Reinigung mit einer äquimolaren Menge Chlorsulfonsäure in Hexan oder n-Dekan bei einer Temperatur von -10°C bis +5°C umsetzt, mit Wasser aufnimmt, mit Xylol extrahiert, mit wässrigem Natriumhydroxid bei einer Temperatur von +50°C bis +70°C neutralisiert, mit Wasserstoff in Gegenwart von Natriumhydroxid bei einem Druck von 1 bis 4 bar und einer Temperatur von +40°C bis +60°C in Gegenwart eines 5%igen Palladium/Kohle-Katalysators dechloriert, durch Umsatz der Mischung mit Phosgen bei einer Temperatur von +95°C in das Sulfonsäurechlorid überführt und anschliessend durch Umsetzung der Reaktionsmischung mit wässriger Ammoniaklösung bei einer Temperatur von +60°C das Endprodukt erzeugt.

## Claims

1. A process for the preparation of 2-(2-chloroethoxy)-benzenesulfonamide of formula I 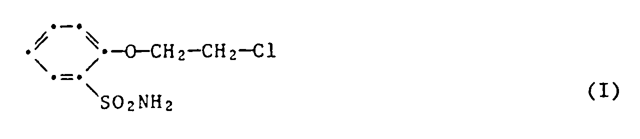 which comprises etherification of 4-chlorophenol of formula II 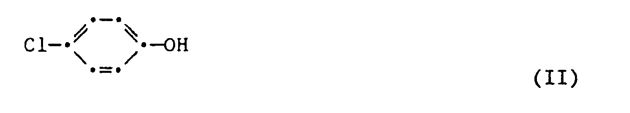 with ethylene carbonate, chlorination of the resulting 4-(2-hydroxyethoxy)-chlorobenzene of formula III 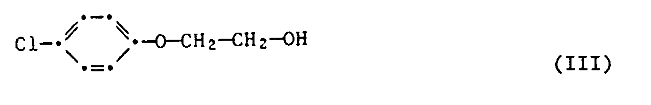 with phosgene or thionyl chloride to give 4-(2-chloroethoxy)-chlorobenzene of formula IV 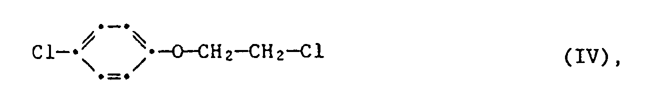 conversion with chlorosulfonic acid ClSO₃H and sodium hydroxide to the sodium sulfonate of formula V 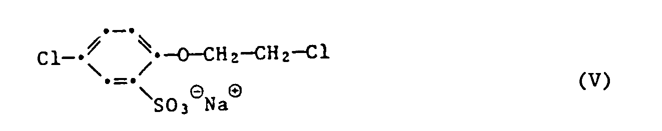 and hydrogenation of this product to give the compound of formula VI 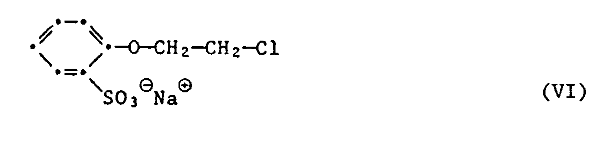 which is subsequently converted with phosgene to the sulfonic acid chloride of formula VII 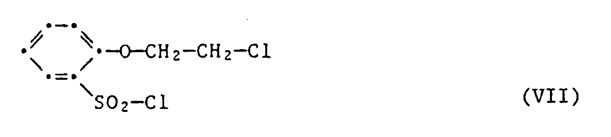 which is reacted with ammonia to give the sulfonamide of formula I.

2. A process according to claim 1, wherein the reaction of the compound of formula II to give the compound of formula IV is carried out, without isolating the intermediate of formula III, by treating the compound of formula II first with ethylene carbonate and subsequently treating the resulting reaction mixture with phosgene or thionyl chloride.

3. A process according to claim 2, wherein the compound of formula II is reacted with ethylene carbonate in the presence of a catalytic amount of tributylamine at a temperature from +130°C to +150°C and the resulting reaction mixture is treated with phosgene or thionyl chloride in the presence of a catalytic amount of dimethylformamide at a temperature from +70°C to +90°C.

4. A process according to claim 1, wherein the reaction of the compound of formula IV to give the compound of formula V is carried out at a temperature from -20°C to +60°C with an equimolar amount of chlorosulfonic acid in hexane or n-decane and the solution is neutralised with aqueous sodium hydroxide at a temperature between +50°C and +90°C.

5. A process according to claim 4, wherein the reaction of the compound of formula IV to give the compound of formula V is carried out at a temperature from -10°C to +5°C with an equimolar amount of chlorosulfonic acid in hexane or n-decane, the solution is taken up in water and extracted with xylene or toluene and then neutralised with aqueous sodium hydroxide at a temperature from +50°C to +70°C.

6. A process according to claim 1, wherein in the course of the conversion of the compound of formula V to the compound of formula VI the dechlorination is carried out with hydrogen at a pressure from 1 to 5 bar and at a temperature from +20°C to +70°C in the presence of a 5% palladium-on-carbon catalyst in a mixture of base and water.

7. A process according to claim 1, wherein the sodium sulfonate of formula VI is converted at a temperature from +60° to +120°C by reaction with phosgene to the sulfonic acid chloride of formula VII.

8. A process according to claim 1, wherein the sulfonic acid chloride of formula VII is reacted with an aqueous solution of ammonia in the presence of chlorobenzene to give the compound of formula I.

9. A process according to claim 1, wherein the reaction of the compound of formula IV to give the compound of formula I is carried out without isolating the intermediates of formulae V, VI and VII.

10. A process according to claim 1, wherein the reaction of the compound of formula II to give the compound of formula I is carried out without isolating the intermediates of formulae III, V, VI and VII.

11. A process according to claim 9, wherein the compound of the formula IV is reacted with an equimolar amount or with a slight excess of chlorosulfonic acid in cyclohexane or n-decane at a temperature from +20°C to +60°C, neutralised with aqueous sodium hydroxide at a temperature from +50°C to +90°C, dechlorinated with hydrogen in the presence of an acid-binding agent at a pressure from 1 to 5 bar and at a temperature from +20°C to +70°C in the presence of a 5% palladium-on-carbon catalyst and converted to the sulfonic acid chloride by reaction of the mixture with phosgene at a temperature from +60°C to +120°C, and subsequently the end product is obtained by reaction of the reaction mixture with aqueous ammonia solution.

12. A process according to claim 11, wherein the compound of formula IV is reacted with an equimolar amount of chlorosulfonic acid in hexane or n-decane at a temperature from -10°C to +5°C, the reaction mixture is taken up in water, extracted with xylene, neutralised with aqueous sodium hydroxide at a temperature from +50°C to +70°C, dechlorinated with hydrogen in the presence of an acid acceptor at a pressure from 1 to 5 bar and at a temperature from +20°C to +70°C in the presence of a 5% palladium-on-carbon catalyst, converted to the sulfonic acid chloride by reaction of the mixture with phosgene at a temperature from +60°C to +120°C, and subsequently the end product is obtained by reaction of the reaction mixture with aqueous ammonia solution.

13. A process according to claim 1, wherein the compound of formula II is reacted with ethylene carbonate in the presence of a catalytic amount of tributylamine at a temperature from +130°C to +150°C, the resulting mixture is converted by reaction with phosgene or thionyl chloride in the presence of a catalytic amount of dimethylformamide at a temperature from +70°C to +90°C to the compound of formula IV, this product after purification is reacted with an equimolar amount of chlorosulfonic acid in hexane or n-decane at a temperature from -10°C to +5°C, neutralised with aqueous sodium hydroxide at a temperature from +50°C to +90°C, dechlorinated with hydrogen in the presence of an acid acceptor at a pressure from 1 to 5 bar and at a temperature from +20°C to +70°C in the presence of a 5% palladium-on-carbon catalyst, converted to the sulfonic acid chloride by reaction of the mixture with phosgene at a temperature from +60°C to +120°C; and subsequently the end product is obtained by reaction of the reaction mixture with aqueous ammonia solution.

14. A process according to claim 13, wherein the compound of formula II is reacted with ethylene carbonate in the presence of a catalytic amount of tributylamine at a temperature from +130°C to +150°C, the resulting mixture is converted by reaction with phosgene or thionyl chloride in the presence of a catalytic amount of dimethylformamide at a temperature from +70°C to 190°C to the compound of formula IV, this product after purification is reacted with an equimolar amount of chlorosulfonic acid in hexane or n-decane at a temperature from -10°C to +5°C; the reaction mixture is taken up in water, extracted with xylene, neutralised with aqueous sodium hydroxide at a temperature from +50°C to +70°C, dechlorinated with hydrogen in the presence of sodium hydroxide at a pressure from 1 to 4 bar and at a temperature from +40°C to +60°C in the presence of a 5% palladium-on-carbon catalyst, converted to the sulfonic acid chloride by reaction of the mixture with phosgene at a temperature of +95°C, and subsequently the end product is obtained by reaction of the reaction mixture with aqueous ammonia solution at a temperature of +60°C.

## Revendications

1. Procédé de préparation du 2-(2-chloréthoxy)-benzosulfonamide de formule I 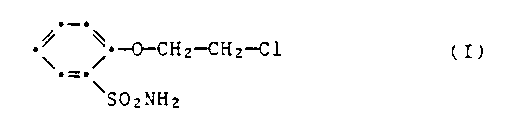 caractérisé en ce que l'on éthérifie un 4-chlorophénol de formule II 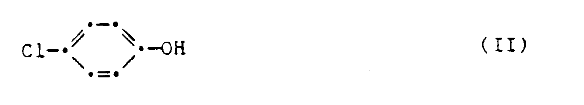 par le carbonate d'éthylène, ce qui donne le 4-(2-hydroxyéthoxy)-chlorobenzène de formule III 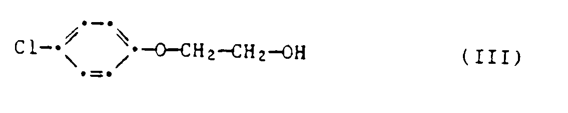 qu'on chlore par le phosgène ou le chlorure de thionyle en le 4-(2-chloréthoxy)-chlorobenzène de formule IV 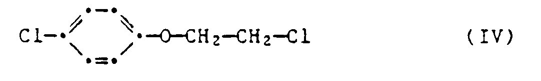 qu'on convertit par l'acide chlorosulfonique ClSO₃H et l'hydroxyde de sodium en le sulfonate de sodium de formule V 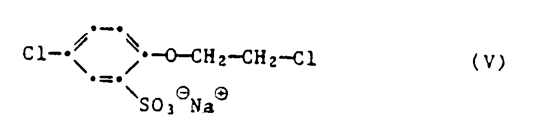 qu'on hydrogène en le composé de formule VI 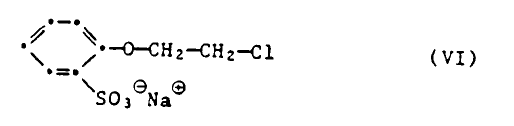 qu'on convertit ensuite par le phosgène en le chlorure d'acide sulfonique de formule VII 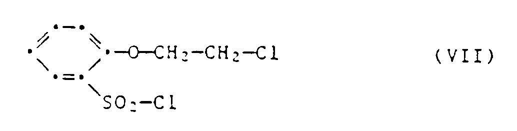 qu'on convertit lui-même à l'aide de l'ammoniac en le sulfonamide de formule I.

2. Procédé selon la revendication 1, caractérisé en ce que la conversion du composé de formule II en le composé de formule IV est réalisée sans isolement du produit intermédiaire de formule III en ce que l'on traite le composé de formule II d'abord par le carbonate d'éthylène puis on traite le mélange de réaction par le phosgène ou le chlorure de thionyle.

3. Procédé selon la revendication 2, caractérisé en ce que l'on fait réagir le composé de formule II avec le carbonate d'éthylène en présence d'une quantité catalytique de tributylamine à une température de +130 à +150°C et on traite le mélange de réaction obtenu par le phosgène ou le chlorure de thionyle en présence d'une quantité catalytique de diméthylformamide à une température de +70 à +90°C.

4. Procédé selon la revendication 1, caractérisé en ce que la conversion du composé de formule IV en le composé de formule V est exécutée à une température de -20 à +60°C à l'aide d'une quantité équimoléculaire d'acide chlorosulfonique dans l'hexane ou le n-décane, et la solution est neutralisée par la lessive de soude à une température de +50 à +90°C.

5. Procédé selon la revendication 4, caractérisé en ce que la conversion du composé de formule IV en le composé de formule V est réalisée à une température de -10 à +5°C à l'aide d'une quantité équimoléculaire d'acide chlorosulfonique dans l'hexane ou le n-décane, la solution est reprise par l'eau, extraite par le xylène ou le toluène puis extraite par la lessive de soude à une température de +50 à +70°C.

6. Procédé selon la revendication 1, caractérisé en ce que, pour la conversion du composé de formule V en le composé de formule VI, la déchloration par l'hydrogène est réalisée sous une pression de 1 à 5 bar et à une température de +20 à +70°C en présence d'un catalyseur consistant en palladium à 5 % sur charbon dans un mélange de gaz et d'eau.

7. Procédé selon la revendication 1, caractérisé en ce que l'on convertit le sulfonate de sodium de formule VI en le chlorure d'acide sulfonique de formule VII par réaction avec le phosgène à une température de +60 à +120°C.

8. Procédé selon la revendication 1, caractérisé en ce que l'on fait réagir le chlorure d'acide sulfonique de formule VII avec de l'ammoniaque en présence de chlorobenzène pour conversion en le composé de formule I.

9. Procédé selon la revendication 1, caractérisé en ce que la conversion du composé de formule IV en le composé de formule I est réalisée sans isolement des produits intermédiaires de formules V, VI et VII.

10. Procédé selon la revendication 1, caractérisé en ce que la conversion du composé de formule II en le composé de formule I est réalisée sans isolement des produits intermédiaires de formules III, V, VI et VII.

11. Procédé selon la revendication 9, caractérisé en ce que l'on fait réagir le composé de formule IV avec une quantité équimoléculaire ou un léger excès d'acide chlorosulfonique dans le cyclohexane ou le n-décane à une température de +20 à +60°C, on neutralise par la lessive de soude à une température de +50 à +90°C, on déchlore par l'hydrogène en présence d'un accepteur d'acide sous une pression de 1 à 5 bar et à une température de +20 à +70°C en présence d'un catalyseur consistant en palladium à 5 % sur charbon, on convertit en le chlorure d'acide sulfonique par réaction du mélange avec le phosgène à une température de +60 à +120°C et on forme ensuite le produit final par réaction du mélange obtenu avec l'ammoniaque.

12. Procédé selon la revendication 11, caractérisé en ce que l'on fait réagir le composé de formule IV avec la quantité équimoléculaire d'acide chlorosulfonique dans l'hexane ou le n-décane à une température de -10 à +5°C, on reprend par l'eau, on extrait par le xylène, on neutralise par la lessive de soude à une température de +50 à +70°C, on déchlore par l'hydrogène en présence d'un accepteur d'acide sous une pression de 1 à 5 bar et à une température de +20 à +70°C en présence d'un catalyseur consistant en palladium à 5 % sur charbon, on convertit en le chlorure d'acide sulfonique par réaction du mélange avec le phosgène à une température de +60 à +120°C puis on forme le produit final par réaction du mélange obtenu avec l'ammoniaque.

13. Procédé selon la revendication 1, caractérisé en ce que l'on fait réagir le composé de formule II avec le carbonate d'éthylène à une température de +130 à +150°C en présence d'une quantité catalytique de tributylamine, on convertit en le composé de formule IV par réaction du mélange obtenu avec le phosgène ou le chlorure de thionyle en présence d'une quantité catalytique de diméthylformamide à une température de +70 à +90°C, on fait réagir le composé de formule IV, après purification, avec une quantité équimoléculaire d'acide chlorosulfonique dans l'hexane ou le n-décane à une température de -10 à +5°C, on neutralise par la lessive de soude à une température de +50 à +90°C, on déchlore par l'hydrogène en présence d'un accepteur d'acide sous une pression de 1 à 5 bar et à une température de +20 à +70°C en présence d'un catalyseur consistant en palladium à 5 % sur charbon, on convertit en le chlorure d'acide sulfonique par réaction du mélange avec le phosgène à une température de +60 à +120°C puis on forme le produit final par réaction du mélange avec l'ammoniaque.

14. Procédé selon la revendication 13, caractérisé en ce que l'on fait réagir le composé de formule II avec le carbonate d'éthylène en présence d'une quantité catalytique de tributylamine à une température de +130 à +150°C, on convertit en le composé de formule IV par réaction du mélange obtenu avec le phosgène ou le chlorure de thionyle en présence d'une quantité catalytique de diméthylformamide à une température de +70 à +90°C, on fait réagir le composé de formule IV, après purification, avec une quantité équimoléculaire d'acide chlorosulfonique dans l'hexane ou le n-décane à une température de -10 à +5°C, on reprend par l'eau, on extrait par le xylène, on neutralise par la lessive de soude à une température de +50 à +70°C, on déchlore par l'hydrogène en présence d'hydroxyde de sodium sous une pression de 1 à 4 bar et à une température de +40 à +60°C en présence d'un catalyseur consistant en palladium à 5 % sur charbon, on convertit en le chlorure d'acide sulfonique par réaction du mélange avec le phosgène à une température de +95°C puis on forme le produit final par réaction du mélange avec l'ammoniaque à une température de +60°C.
